# EUROPEAN PATENT APPLICATION

(11) **EP 4 588 418 A1**
(43) Date of publication of application: **23.07.2025**
(21) Application number: 24152070.9
(22) Date of filing: 16.01.2024
(51) Int. Cl.: A61B 1/00, A61B 1/005, A61B 1/05, A61B 1/313, A61B 1/317

(54) **A SOFT ROBOTIC IMAGING DEVICE, AN IMAGING SYSTEM FOR MINIMALLY INVASIVE SURGICAL PROCEDURES AND A CONTROLLING METHOD OF THE SOFT ROBOTIC IMAGING DEVICE AND THE IMAGING SYSTEM**

(71) Applicant: Asvel OÜ, 76702 Laulasmaa küla (EE)
(72) Inventor: Savel, Andres, 11313 Tallinn (EE); Aabloo, Alvo, 68613 Patküla (EE); Soni, Ritesh, 50407 Tartu (EE)
(74) Representative: Moosedog Oy

(57) **Abstract**

A soft robotic imaging device (100) comprising a partially extendable scope with a soft robotic actuated flexible distal part (114) that can bend in various directions. The device comprises an imaging means (116) at its distal end to capture and transmit video imagery during surgery. Additionally, it comprises a surgical access device (120) with a detachable fluid channel (130) and a fixation mechanism (140). The electronic hardware integrated into the system is housed within a handle (150) attached to the proximal part of the scope. The soft robotic actuated flexible distal part may be constructed from one or more conductive polymers. The device is designed for easy integration into an imaging system for minimally invasive surgical procedures. The system aids in detecting surgical instruments, monitoring tissue conditions, and tracking instrument movements and allows the surgeon to synchronize the flexible distal part's movements with surgical instruments and voice command-based features to provides surgeons with enhanced visibility and control.

## Description

### TECHNICAL FIELD

The present disclosure relates generally to the field of medical devices, and more specifically endoscopes for various minimally invasive surgical (MIS) procedures.

### BACKGROUND

Minimally invasive surgery (MIS) procedures, also known as "key-hole" surgeries, have revolutionized healthcare by minimizing surgery-related trauma and enhancing patient outcomes. Over 17 million of these procedures, including laparoscopy, arthroscopy, endoscopic surgery, and others, are performed annually worldwide. These techniques employ medical scopes with charged-coupled device (CCD) cameras, inserted through small incisions or natural body openings, to observe internal anatomical areas during surgery. However, these procedures face significant challenges. The manual control of scopes demands high surgeon dexterity and often requires additional personnel for camera manipulation, posing risks of internal tissue damage and patient complications due to prolonged operation times.

Current medical scopes used in MIS, such as traditional endoscopes (arthroscope, laparoscope, thoracoscope, etc.), have a design largely unchanged for over a century. They typically consist of a straight tube housing lenses at various angles, a light source, and other optical components, with improvements like optical cable illumination and camera integration enabling real-time visualization and remote sharing. The rigidity of these scopes limits the field of view, potentially leaving some diseases or injuries undiagnosed. The surgeon has to manually manipulate the scope, holding it with one hand and operating surgical instruments with the other. In complex surgeries, an assistant is often needed to position the scope, affecting the surgeon's ability to quickly and effectively perform operations.

Such known devices used in minimally invasive endoscopic operations across various medical fields are often partially automated large machines. These solutions have several drawbacks. They often rely on a rigid endoscope, limiting visibility and maneuverability. Their substantial size, high cost, and maintenance expenses, coupled with the complexity of training, limit their accessibility and suitability for all procedures. Alternative devices allowing manual bending of the endoscope's tip have been developed, but they still suffer from limitations like large diameter and heavy weight, making them unsuitable for certain operations like arthroscopy.

The known medical scopes and minimally invasive surgery (MIS) techniques are associated with a range of problems and disadvantages. Surgeons must manually manipulate the medical scope, which require specific skills and often additional personnel for camera manipulation. Manual control and movement of the scope inside the body can cause damage to internal tissues, organs, arteries, etc. The manual use of the scope increases the risk of complications such as bleeding, infection, and prolonged operation times, which could lead to issues like compartment syndrome. The rigidity of traditional scopes restricts the surgeon's field of view and hinders visibility around corners, potentially leaving some diseases or injuries undiagnosed. Holding the scope with one hand limits the surgeon's ability to perform other necessary tasks during surgery. Complex operations often require an assistant to hold the camera, which can impact the efficiency and effectiveness of the surgery, especially if the assistant is inexperienced. Partially automated solutions like large robotic systems are expensive, occupy significant space, and incur substantial annual maintenance expenses. Surgeons require extensive training to operate automated systems, which can be time-consuming and complex. Some automated systems with rigid endoscopes are not suitable for all types of endoscopic procedures, such as arthroscopy. Current medical scopes offer a two-dimensional view and are difficult to maneuver compared to traditional open surgical procedures.

Given these challenges, there is a need for more compact, autonomous, and cost-effective medical scopes that would overcome the limitations of traditional and existing endoscopic systems.

### SUMMARY

The aim of the present disclosure is to provide a soft robotic imaging device, an imaging system for minimally invasive surgical procedures and a controlling method of the soft robotic imaging device and the imaging system to free the surgeon or assistant from the job of holding the camera with one hand so that the surgeon or assistant can operate with two hands and to improve visibility of the surgeons during the MIS procedures.

The aim of the disclosure is achieved by the soft robotic imaging device, imaging system and a controlling method of the soft robotic imaging device and the imaging system as defined in the appended independent claims to which reference is made to. Advantageous features are set out in the appended dependent claims.

The advantage of the embodiments of the present disclosure is that the hands-free automatically controllable flexible soft robotic imaging device provides to the surgeon better visibility during the surgical procedures and thus the procedures are more precise, faster and there are less complications. Additional aspects, advantages, features and objects of the present disclosure would be made apparent from the drawings and the detailed description of the illustrative embodiments construed in conjunction with the appended claims that follow.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the embodiments of the disclosure are shown in the drawings, with references to the following diagrams wherein:
Fig. 1a is a front view of a soft robotic imaging device for minimally invasive surgical procedure according to an embodiment of the present disclosure;
Fig. 1b is a top view of the soft robotic imaging device shown in Fig. 1a;
Fig. 2 is a front view of an imaging system for minimally invasive surgical procedure;
Fig. 3a and Fig. 3b illustrate a soft robotic actuated flexible distal part of a scope of the soft robotic imaging device shown in Fig. 1a and 1b;
Fig. 4 is a front view of a soft robotic imaging device for minimally invasive surgical procedures according to another embodiment of the present disclosure.

### DETAILED DESCRIPTION OF EMBODIMENTS

The following detailed description illustrates embodiments of the present disclosure and ways in which they can be implemented. In an aspect, an embodiment of the present disclosure provides a soft robotic imaging device for a minimally invasive surgical procedure, the soft robotic imaging device comprises a scope arranged to be at least partially extendable and having a proximal part and a soft robotic actuated flexible distal part adapted to bend in multiple directions; an imaging means positioned at an end of the flexible distal part of the scope; a surgical access device comprising a fluid channel and is detachably connectable with the proximal part of the scope; a fixation mechanism of the surgical access device; and an integrated electronic hardware.

The soft robotic imaging device for minimally invasive surgical procedure enables to solve the challenges associated with traditional surgical scopes, including limited visibility, maneuverability, and the need for manual adjustments. The soft robotic imaging device's capabilities empower surgeons with advanced tools for performing MIS procedures with greater precision, safety, and efficiency, all driven by automated computational control system algorithms.

The soft robotic imaging device enables autonomous tracking of surgical equipment within the body cavities and frees the surgeon from manual control of the soft robotic imaging device by utilizing flexible, biocompatible conductive polymer materials based microactuators for automated computer-controlled system algorithms guided image tracking and voice-controlled movements. The soft robotic imaging device thus enables to transform MIS procedures to a new level by simplifying the surgeon's work and reducing operation time, complications, number of assisting personnel number and improving patient care through advanced technology and expertise. The construction of the soft robotic imaging device makes it suitable for various types of MIS procedures (e.g. arthroscopy, laparoscopy, thoracoscopy, veterinary MIS procedures), and is more durable, lighter and smaller than known endoscopic systems.

The soft robotic imaging device is arranged to navigate autonomously, adapting to the complexities of tissue structures that were previously challenging to access with conventional devices. As a result, surgeons gain the ability to operate more efficiently with both hands, enhancing their overall control and precision during surgical procedures.

The scope is arranged to be at least partially extendable and has a proximal part and a soft robotic actuated flexible distal part adapted to bend in multiple directions has two ends, at one end there is an imaging means (e.g., an image sensor or micro camera) attached, the second end is connected with the proximal part forming at the connection point an anchored end. Flexibility of the scope enables it to bend around the anchored end. Further, the soft robotic actuated flexible distal part of the scope may be formed of multiple segments. In the examples of the soft robotic actuated flexible distal part of the scope having a cross-leaf structure, the soft robotic actuated flexible distal part comprises multiple segments. Each segment in the cross-leaf structure is arranged to bend and rotate independently. This enables to turn the distal end with the camera to any direction, which allows the camera to capture a comprehensive view of its entire surroundings.

The imaging means positioned at an end of the flexible distal part of the scope integrated into the soft robotic imaging device is crucial for providing a clear vision and a live video feed of the surgical operation area. It supports automated computational controlled system-driven surgical equipment tracking and ensures real-time monitoring. The integration of the soft robotic imaging device is essential as it provides clear vision and live video feed of the surgical operation area, serving as the foundation for image tracking and real-time monitoring. This key component significantly contributes to accurate visualization, enabling the tracking of surgical instruments and facilitating dynamic adjustments to the device's position during procedures. The imaging means holds crucial advantages for surgical interventions. It elevates the surgeon's situational awareness, leading to more informed decision-making. By offering an enhanced view, it plays a pivotal role in the success of minimally invasive procedures.

Importantly, it effectively resolves the longstanding issue of limited visibility associated with traditional scopes. Additionally, the integration of a micro camera and the consequential size reduction not only improves the device's maneuverability but also aligns with a less invasive approach to surgeries. This dual benefit not only enhances precision but also contributes to a reduction in wound trauma, fostering improved patient outcomes.

The imaging means are configured to capture and transmit video imagery of surrounding tissues during the minimally invasive surgical procedure. The imaging means may be for example one or more image sensors or micro camera. The micro camera with advanced image sensor technology to reduce the diameter or size of the scope (less than 2 mm) and to provide a clear vision and live video feed of inside the body during MIS procedures to the surgeon is preferred. This micro camera size (diameter is less than 2 mm) is in the range of 0.2 mm to 2 mm (length) and 0.2 mm to 2 mm (width), with built in light source. To make the system more versatile, it incorporates automated computer-controlled system algorithms based on machine vision techniques, such as instance segmentation, to recognize equipment and key features of its surroundings without the need for physical markers. This advanced approach translates pixel-level recognition into precise positional coordinates in 3D. The automated computational control system algorithms at the soft robotic imaging device are configured to use this positional information to synchronize its movements with surgical equipment, enhancing precision and safety in surgical procedures.

The surgical access device is for creating an access point, for supporting the proximal part of the scope when the proximal part of the scope is connected to the surgical access device and for conducting fluids (i.e. solutions or gasses). The surgical access device can be e.g., a trocar with obturator, a laparoscopic access device, a surgical port, an endoscopic access system, a single-port surgery device.

In a preferred embodiment, the surgical access device is a trocar comprising at one end a hollow sleeve for inserting it into body cavity and on the other end is a handle. The handle comprises automatic locking mechanism for fixation of a scope (i.e. arthroscope, laparoscope, thoracoscope) or optionally an obturator into the trocar. For releasing the obturator or the scope from trocar, there are releasing button on trocar. Trocar handle comprises also outer fluid channels or gas channel with taps from which the fluid (physiological solution) e.g. for arthroscopy or gas (carbon dioxide) e.g. for laparoscopy leaded into the body cavity through trocar hollow sleeve and between scope inside trocar. This trocar with fluid or gas channel is particularly useful in procedures where controlled delivery of fluids or gas into the body cavity is necessary, enhancing both the safety and efficiency of the surgical procedure.

The obturator with a blunt end is inserted into the hollow sleeve of the trocar, with which the trocar can be safely inserted into the body cavity through tiny skin incision and perforating subcutaneous tissues bluntly for avoiding damages for subcutaneous nerves or vessels. As the trocar is inserted into the body cavity, the obturator is removed from the trocar by using the releasing button on trocar and scope is inserted through the trocar into the body cavity.

The trocar fluid or gas channel comprises an inlet compatible with medical tubing, a connection mechanism designed to securely attach the fluid hose to the trocar. The connection mechanism ensures a tight, leak-proof seal with the hollow tube of the trocar and allows for easy attachment and detachment. The fluid channel may optionally comprise an outlet. The fluid or gas channel may further comprise a flow control mechanism for enabling to control of the rate and volume of fluid entering the body cavity, which is crucial for patient safety and the effectiveness of the procedure.

The inlet and optionally the outlet of the fluid channels are adjusted for directing fluids, such as physiological solution, to the surgical operation area during surgical procedures. The fluid inlet and outlet system is a crucial component in the present soft robotic imaging device used for various MIS procedures, such as arthroscopic or endoscopic surgeries. The fluid channel involves the delivery of fluid from fluid container through an external sterile hose, which passes through the trocar sleeve and between the scope distal part. The fluid is then discharged into the joint or body cavity at the end of trocar. This process serves several important functions during surgical procedures.

The physiological solution is used to distend the joint or body cavity to create a working space for the surgeon and facilitates better visualization of internal structures. Also it is used as an irrigating fluid to clear and cleanse the surgical operation area, providing a clearer view for the medical professional operating the scope. This is particularly important in arthroscopic procedures where joint spaces need to be visualized. For laparoscopy, the introduction of gas (e. g. carbon dioxide) into the intraperitoneal room helps to distend it and create a pneumoperitoneum.

The outer commonly used equipment system for physiological solution or gas pressure allows control over the pressure within the joint or body cavity. This is important to prevent the collapse of the space being examined and to ensure a stable environment for surgical or diagnostic procedures. In some procedures, the use of gas allows for improved visualization and manipulation. For example, in laparoscopic surgery, carbon dioxide gas is commonly used to create a pneumoperitoneum, providing a clear field of view. The design of the fluid channel ensures that the fluid or gas is delivered in a controlled and sterile manner, minimizing the risk of contamination. The system's efficiency directly impacts the success and safety of the surgical procedure, as it influences the surgeon's ability to visualize and perform tasks within the body cavity or joint spaces.

The fixation mechanism of the surgical access device is for holding the surgical access device (trocar) on the skin. The fixation device can be e.g. solution resistant adhesive patch having a central opening for a trocar with a seal or collar, or a gelport to hold trocar. In a preferred embodiment, the adhesive patch is used, which may be a solution resistant adhesive patch. The adhesive patch comprises a central tiltable collar around the central opening of the adhesive patch, forming a tiltable part of the patch between the collar and the patch, to provide more secure support for the trocar when trocar is tilted during the procedure. The trocar can tightly slide inside the collar of the adhesive patch. The fixation mechanism ensures optimal positioning of the scope, allowing for an enhanced and precise view of the surgical operation area. By providing stable fixation, the device minimizes the need for manual adjustments during surgical procedures. The fixation mechanism ensures stable fixation of the soft robotic imaging device, enhancing the precision of the surgical procedure.

The integrated electronic hardware may comprise a microprocessor selected from a group comprising a vision processing unit (VPU), computer vision processor (CVP) chip, system-on-chip (SOC) or Complementary Metal-Oxide-Semiconductor (CMOS) chip. To tackle the integration challenges of emanating wires from external control and thereby aim for a miniaturized energy-efficient solution, a computer vision processor (CVP) chip using fast, low-power analog may be used. The live images are transferred to a simple on-chip control to control movements of the scope and to show the surgical operation area.

Alternatively, an energy-efficient SOC through a CMOS Chip Design with Image-Guided automated computer-controlled system algorithms and with built-in control may be used. The CMOS chip design methods for soft robotic imaging device, emphasizing energy efficiency, miniaturization, and embedded control functions enables to enhance device precision and maneuverability, ultimately advancing medical technology, and patient care. The SoC architecture for soft robotic imaging device, integrating real-time computer-controlled algorithms, sensor interfaces, and communication protocols ensures a precise and seamless scope movement control.

The built-in control, adjacent to the micro-camera, to control the microactuators movement based on live video imaging while keeping its size and energy consumption low. The micro-camera that generates a voltage corresponding to the light intensity accumulated in the pixel array requires additional processing and control to guide the microactuators.

For accommodating the electronic hardware, the soft robotic imaging device may comprise a handle attached to the end of the proximal part of the scope arranged to accommodate the integrated electronic hardware. The handle may have a wired or wireless connection to one or more computing devices.

The soft robotic actuated flexible distal part may be constructed using one or more conductive polymers. The one or more conductive polymers may be selected from a group comprising polypyrrole (PPy), poly(3,4-ethylenedioxythiophene) polystyrene sulfonate (PEDOT: PSS), polyaniline, polythiophene or polyacetylene, poly(3-hexylthiophene) (P3HT).

Such biocompatible, flexible, adaptable, soft materials in the present soft robotic imaging device enable enhanced maneuverability and reduced tissue damage during insertion and navigation during MIS procedures. These conductive polymers enable to provide flexibility to the scope. This flexibility enables the scope to move in all directions, providing a three-dimensional (3D) view during MIS procedures. The flexibility of the scope is essential, serving as a foundational element that enables the device to navigate through the body with minimal invasiveness during Minimally Invasive Surgical (MIS) procedures.

The utilization of soft and biocompatible materials, specifically conductive polymers (CPs), in the construction ensures the safety and compatibility of the device with internal tissues. The flexibility of the scope provides a three-dimensional view of the internal body during surgical procedure. By offering flexibility, it empowers surgeons with enhanced visibility and maneuverability, enabling them to navigate through intricate anatomical structures with precision.

The advantages of a flexible scope are multifaceted. Firstly, it significantly reduces tissue damage (e.g. smaller wounds, less internal organ damage), a critical factor in promoting faster recovery and minimizing postoperative complications. Secondly, the flexibility of the scope improves surgical precision, allowing for more accurate and targeted interventions. Lastly, it facilitates the performance of more complex procedures in confined body spaces, which is particularly challenging with traditional rigid scopes. In essence, this feature addresses the longstanding issues of limited visibility and maneuverability associated with rigid scopes, ushering in a new era of sophistication and efficacy in surgical procedures.

The flexible distal part may be cross-leaf structured with notches, chain-like structured, tentacle-structured, tube-structured, sheet-structured, amorphous or muscle-like. The cross-leaf structured segments made of conductive polymers act also as soft microactuators which enable scope's movement in all directions. Additionally, by using micro-springs at the end of the actuators it is also possible to automatically extend or contract the length of the scope which with the help of micro camera during live video imaging further provides a three-dimensional (3D) view of the surgical operation area.

The soft robotic cross-leaf structured microactuators are necessary to drive the movement of the flexible scope. These actuators are adapted to be capable of responding to automated computationally controlled system-driven commands for precise control. The soft robotic cross-leaf structured microactuators assume a pivotal role in propelling the movement of the flexible scope, acting as the driving force behind its locomotion and promptly responding to commands for meticulous control. These cross-leaf structured microactuators bring a dynamic and controlled dimension to the movement of the scope. Their responsiveness facilitates real-time adjustments tailored to the specific requirements of the ongoing surgical procedure, ensuring optimal positioning and adaptability. The incorporation of cross-leaf structured microactuators substantially enhances adaptability and responsiveness, allowing seamless navigation and precise control. This facilitates automated and streamlined surgical processes, addressing the challenge of manual adjustments. Additionally, microactuators contribute to a noteworthy reduction in device size, enhancing maneuverability in confined spaces and advancing minimally invasive surgical techniques. Importantly, the smaller scope size due to microactuators plays a pivotal role in minimizing wound trauma during surgical interventions. This allows for gentler entry and movement within the body, lowering the impact on tissues and promoting a less invasive and patient-friendly surgical approach-advancing comfort and outcomes in minimally invasive surgeries.

The soft robotic actuated flexible distal part may comprise biocompatible encapsulation. Ensuring biocompatibility is of the utmost importance when introducing the soft robotic imaging device into the body during surgical procedures. To achieve this, a comprehensive encapsulation of the scope using a biocompatible polymer-based solution is coated by dip coating method. This biocompatible encapsulation coating is for the protection of scope from body fluids and also prevents this scope from interacting with the inside body's environment during MIS procedures. The coating safeguards the scope of the soft robotic imaging device and prevent it from interacting with the inside body's environment. To ensure biocompatibility, the soft robotic imaging device requires a protective coating. This encapsulation prevents interaction with the inside of the body environment and also protects the scope. Encapsulation with a biocompatible material is crucial to prevent adverse interactions between the device and the body's internal environment. This provides a protective barrier, serving as a shield against damage to internal tissues and significantly reducing the risk of complications during and after surgical procedures.

The biocompatible encapsulation significantly enhances the safety profile of the device. By forming a protective barrier, it minimizes the potential for infections or adverse reactions, ensuring harmonious compatibility with the human body. This encapsulation solution effectively solves the problem of potential harm caused by the device's interaction with bodily tissues, promoting a secure and reliable surgical tool. Moreover, it aligns with the broader goal of improving patient outcomes and safety in medical interventions by mitigating the risks associated with device implantation or interaction within the body.

The fixation mechanism may be constructed of a biocompatible and antiallergic adhesive polymer. The fixation mechanism, adhesive patch to hold a trocar, for fixation the device to human body, is a pivotal component designed to secure the device to the human body, particularly through a skin wound. Crafted from an adhesive polymer, this ensures stable fixation, optimizing the performance and accuracy of the device during surgical procedures. Even in scenarios where the device is not adequately stabilized, automated computational control system algorithms-guided actuators intervene to maintain a stable camera view, enhancing the overall effectiveness of the device.

The fixation mechanism is constructed from a specialized adhesive polymer known for its biocompatibility and skin-safe properties. The adhesive nature of the polymer allows the device to securely adhere to the skin, creating a stable connection point for the soft robotic imaging device. A trocar facilitates the insertion of the soft robotic imaging device through the wound point.

Such design of the fixation mechanism minimizes trauma during insertion, contributing to a less invasive approach to surgical procedures. The fixation mechanism represents a critical element in the soft robotic imaging device, addressing the need for secure attachment to the human body while optimizing stability and precision during minimally invasive surgeries.

The imaging means may comprise one or more of a light source, an anti-fog coating, one or more additional sensors. The integration of an imaging means and light source with soft robotic microactuators allow it to serve as an internal body visualization tool in multiple directions. Under the automated computer-controlled system algorithms guidance, this scope inside the body cavities can change its position based on the surgeon's surgical equipment movements, eliminating the need for manual adjustments. The automated computer-controlled system algorithms 's ability to track surgical equipment movements and voice commands, empowering surgeons to work with both hands and freeing the hands from camera management duties.

In another aspect, an embodiment of the present disclosure provides an imaging system for minimally invasive surgical procedures, wherein the system comprises an imaging device according to any of the embodiments of the present disclosure; a computing device comprising an automated control system of the imaging device arranged to receive and process imagery from the imaging device; a hands-free control system of the imaging device; an imagery display device arranged to display the imagery received from the imaging device; a first connectivity interface configured to establish communication between the imaging device and the computing device; and a second connectivity interface configured to establish communication between the hands-free control system and the imaging device.

The hands-free control system may be e.g., a voice recognition and command system using a laryngophone, which enables human-soft robotic imaging device interaction (HSRI). These voice commands operate on computational control system algorithms, designed to activate, direct, and guide the movements of the soft robotic actuated flexible distal part of the soft robotic imaging device, such as moving left, right, up, down, zooming in or out, or stop to achieve optimal observation of specific internal areas within the body. The precision of these voice commands extends to dictating specific angles of movement, including 30°, 45°, 90°, or 180° rotations. This level of control allows the surgeon to optimize the observation of particular internal areas within the body during MIS procedures. By incorporating this feature, surgeons gain precise external control over the soft robotic imaging device's movements, enhancing overall precision and maneuverability. By employing this feature, precise external control over movements of the soft robotic imaging device enables the surgeon to enhance overall precision and maneuverability. This enhancement in HSRI represents a significant advancement in the field of medical research.

In tandem with the automated machine vision techniques-guided surgical equipment tracking, additional advanced functionality is introduced to enhance HSRI between the surgeon and the scope of the soft robotic imaging device -for instance voice-based commands issued by the surgeon via laryngophone.

Voice-command automated computational control system algorithms enable human-softrobot interaction, allowing surgeons to control the device via a laryngophone. This feature enhances overall precision and maneuverability. The integration of voice-command automated computational control system algorithms assumes critical importance by empowering surgeons to control the device seamlessly through a laryngophone, thereby enhancing human-softrobot interaction. This feature contributes significantly by enabling hands-free control of the device. Surgeons can issue commands through voice, eliminating the need for manual adjustments during procedures. This hands-free capability enhances the overall efficiency of the surgical process.

The implementation of voice-command automated computational control system algorithms brings forth several noteworthy advantages. Firstly, it improves the overall maneuverability of the device, allowing surgeons to navigate and position the scope with greater ease. Hands-free control not only streamlines the surgical workflow but also enhances responsiveness during critical moments of the procedure. This innovation addresses a longstanding challenge associated with traditional scopes, which often require manual adjustments. By offering more intuitive and hands-free interaction, surgeons can concentrate more on the surgical task at hand, fostering improved focus and precision. This feature represents a significant leap in user experience and marks a considerable advancement in the field of MIS procedures.

The automated control system is arranged, based on the processed imagery, to detect surgical instruments; detect and visualise surgical operation area; monitor condition of surrounding tissues and upon detection of tissue damages to generate an alert related to the detected tissue damages; track movements of surgical instruments during the minimally invasive surgical procedure; synchronize the movements with surgical instruments. The hands-free control system is arranged to trigger, and to guide actions of the soft robotic imaging device. The soft robotic imaging device is equipped with an advanced voice command feature, allowing surgeons to exert precise control over its flexible distal part using a laryngophone during MIS procedure. This innovative technology enables the surgeon to issue verbal commands, directing the scope to move in multiple directions and facilitating seamless zoom in/out operations for enhanced precision and better visibility.

The automated control system by utilizing the flexible design of the scope further enables to dynamic monitoring of the real-time movements of surgical equipment, accompanied by live video streaming of the surgical region which offers a clear and up-to-the-minute view during surgery. The scope employs a dual approach to surgical equipment tracking: initially relying on an automated computational object tracking algorithm that follows a distinct fiducial marker attached to surgical equipment, such as color patterns or alphabet characters.

The system further incorporates automated computation vision techniques, such as instance segmentation, to recognize surgical equipment and key features of its surroundings (e.g., joint cartilage, synovia, tendons, vessels, peritoneum, and intestines) without the need for physical markers. Also, the automated computer controlled system enables to detection of any injured and bleeding vessel to inform the surgeon about potential risks thus helping to reduce risks more quickly. This automated computer-controlled system algorithms at the core of scope then uses this positional information to synchronize its movements with surgical equipment, enhancing precision and safety during surgical procedures. The scope's capabilities in live video and data recording are invaluable for improving training and advancing medical research, marking a groundbreaking advancement in MIS procedures.

The automated computational control system algorithms are essential for accurately detecting and tracking surgical equipment in real time. This feature enhances the surgeon's view during MIS procedures. The utilization of automated computational control system algorithms is pivotal for the accurate detection and real-time tracking of surgical equipment, significantly enhancing the surgeon's view during surgical procedures. This advanced tracking system contributes by providing precise and dynamic tracking of surgical instruments. It establishes seamless coordination between the device's movements and the ongoing surgical actions, ensuring optimal positioning and alignment.

The incorporation of automated computational control system algorithms provides several advantages. Firstly, it enhances surgical precision by enabling the device to accurately follow and track surgical instruments in real time. This heightened precision, in turn, reduces the risk of errors and complications during minimally invasive surgeries, ultimately contributing to successful outcomes. Moreover, this sophisticated tracking system effectively addresses the long-standing challenge of limited tracking capabilities associated with traditional scopes. By providing a comprehensive and real-time view of surgical instruments, it overcomes the constraints of manual tracking, offering a more automated and reliable solution. This advancement aligns with the overarching objective of improving the efficacy and safety of surgical interventions, marking a significant step forward in the field of minimally invasive procedures.

The soft robotic imaging device features automated computational control system algorithms-guided actuators that intervene when the scope body encounters any instability. The algorithms continuously monitor the position of the scope of the soft robotic imaging device and, when necessary, autonomously adjust the positioning of the scope to maintain a stable camera view.

In a third aspect, the present disclosure provides a controlling method of a soft robotic imaging device and an imaging system for minimally invasive surgical procedure, wherein the method comprises steps of receiving, in a computing device, imagery from imaging means positioned at an end of a flexible distal part of scope at a site of the minimally invasive surgical procedure; collecting data by the computing device from the received imagery by detecting surgical instruments, detecting features of surroundings of the surgical instruments, detecting and visualising surgical operation area, monitoring condition of surrounding tissues and upon detection of tissue damages generating an alert related to the detected tissue damages, and tracking movements of surgical instruments during the MIS procedure; based on the collected data, synchronizing movements of the flexible distal part of the scope with surgical instruments.

According to the embodiments, synchronizing the movements comprises applying voltage to the soft robotic actuated flexible distal part of the scope. Applying voltage to the soft robotic actuated flexible distal part of the scope enables to move the end of the distal part of the scope and direct the camera at the end of the distal part to the desired direction. In combination with surgeon's voice commands the integration of a micro camera with microactuators allows the soft robotic imaging device to serve as an internal body visualization tool. Under computer-controlled algorithms, the soft robotic imaging device enables to change its position based on the surgeon's voice commands, eliminating the need for manual adjustments and enabling surgeons to work with both hands and freeing them from camera management duties. In addition, the movement of the flexible arm of the present scope is automated to visualize the precisely operated area with the microcamera and track the surgical equipment and also can move according to given voice command by the surgeon thanks to the automated computer-controlled system algorithms.

### DETAILED DESCRIPTION OF THE DRAWINGS

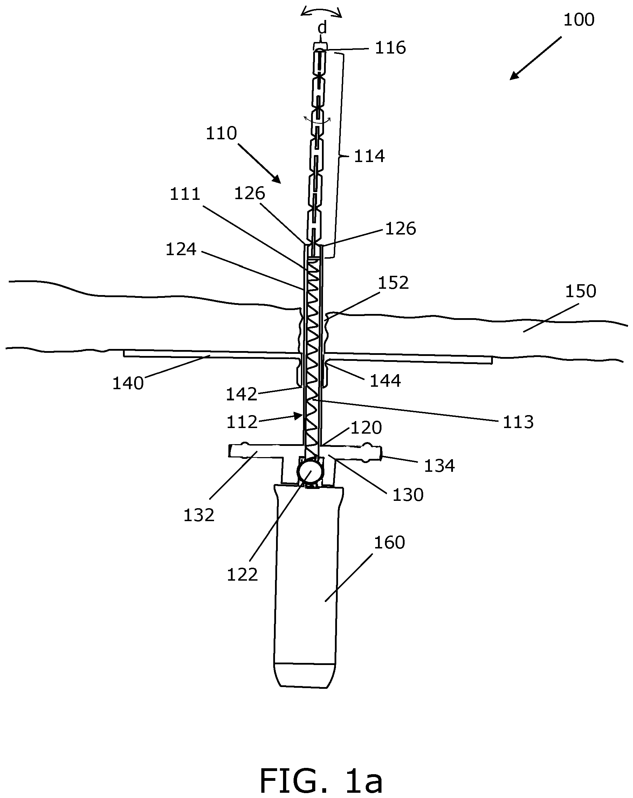

Referring to Fig. 1a, there is shown a front view of a soft robotic imaging device 100 for minimally invasive surgical procedure according to an embodiment of the present disclosure. The soft robotic imaging device 100 is inserted through a small incision 152 of the skin 150. The soft robotic imaging device 100 for minimally invasive surgical procedure comprises a scope 110 arranged to be at least partially extendable and having a proximal part 112 and a soft robotic actuated flexible distal part 114 adapted to bend in multiple directions, an a camera 116 with a light source positioned at an end of the flexible distal part of the scope, an arthroscopic trocar 120 comprising a fluid channel 130 and is detachably connectable with the proximal part 120 of the scope 110, a solution resistant adhesive patch 140 of the surgical access device to hold the trocar **120** on a skin **150** and an handle 160 attached to the end of the proximal part of the scope and arranged to accommodate the integrated electronic hardware.

The scope 110 comprises scope sleeve 111. The proximal part 112 of the scope comprises a contracting and extensible micro-spring 113, shown in extended position. The soft robotic actuated flexible distal part 114 is in the form of cross-leaf structured flexible actuator with notches. The trocar 120 further comprises a trocar release button 122 and a trocar sleeve 124 surrounding a scope sleeve 111. Between trocar sleeve 124 and the scope sleeve 111, there is formed a fluid channel 126 around the scope sleeve 111. The fluid channel 130 comprises an inlet 132 and an outlet 134. The solution resistant adhesive patch 140 comprises a central tiltable collar 142 around the central opening of the adhesive patch, forming a tiltable part 144 of the patch between the collar and the patch.

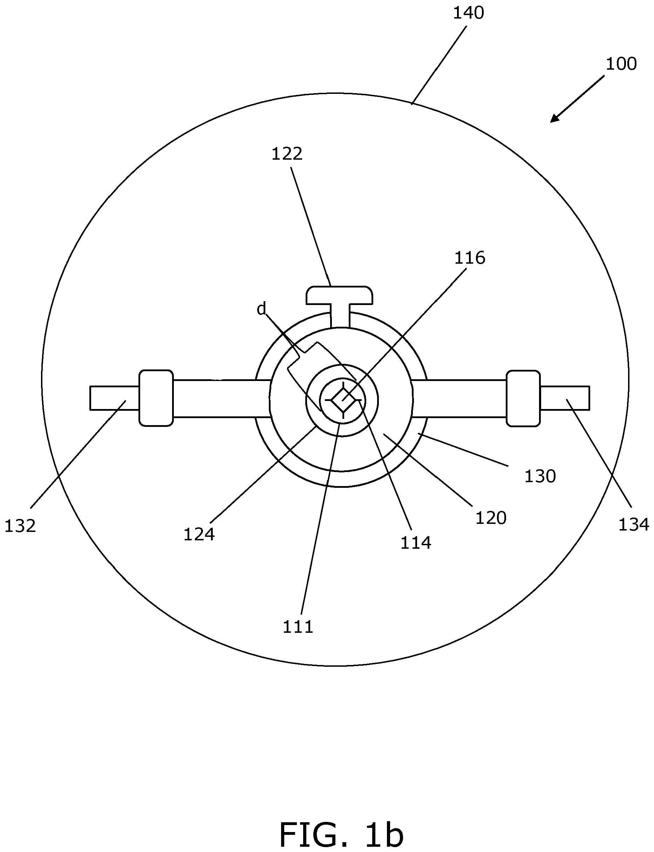

Referring to Fig. 1b, there is shown a top view from distal end of the soft robotic imaging device 100 shown in Fig. 1a. The top view of the soft robotic imaging device **100** shows the soft robotic actuated flexible distal part **114,** the camera **116** with a light source positioned at the end of the flexible distal part of the scope, the arthroscopic trocar 120 comprising the fluid channel **130,** the solution resistant adhesive patch **140,** the trocar release button **122,** the trocar sleeve **124** surrounding a scope sleeve **111,** the scope sleeve **111,** the inlet **132** and the outlet **134** of the fluid channel **130.**

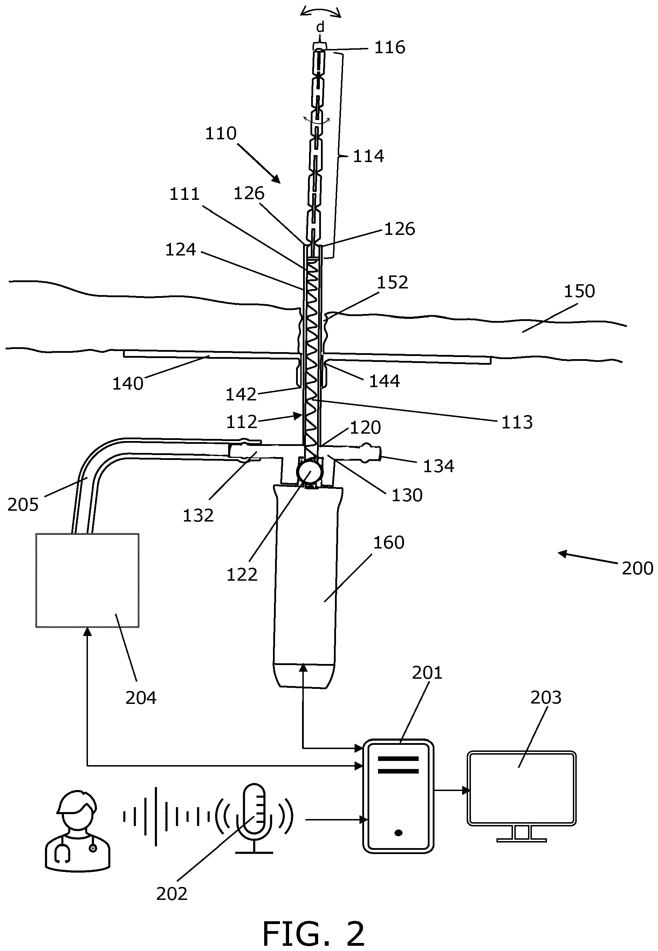

Referring to Fig. 2, there is shown a front view of an imaging system **200** for minimally invasive surgical procedure. The imaging system 200 comprises a soft robotic imaging device **100** shown in figures Fig. 1a and Fig. 1b, a computing device **201,** hands-free control system of the soft robotic imaging device **202,** an imagery display device **203** and a pump **204** of a physiological pressure control, which is connected to an inlet **132** of the trocar **120** by a hose **205.**

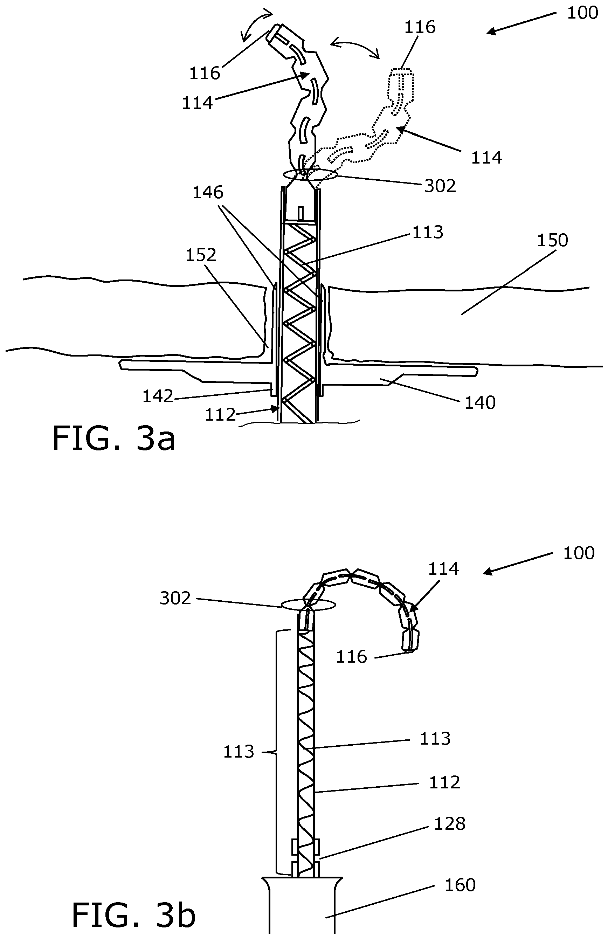

Referring to Fig. 3a and Fig. 3b, there is illustrated the soft robotic actuated flexible distal part of the scope of the soft robotic imaging device shown in Fig. 1a and inf Fig. 1b. The figures Fig. 3a and Fig. 3b illustrate the multidirectional bendability of the soft robotic actuated flexible distal part **114** of the scope. The soft robotic actuated flexible distal part of the scope has two ends, at one end there is an imaging device (e.g., a camera) 116 attached, the second end is connected with the proximal part forming at the connection point and bending point **302.** The flexibility of the scope enables it to bend around the bending point **302.** The soft robotic actuated flexible distal part of the scope comprises multiple segments. Each segment in the cross-leaf structure is arranged to bend and rotate independently.

Figure Fig. 3a illustrates the omnidirectional movement of the soft robotic actuated flexible distal part of the scope which is inserted through a small incision **152** of the skin **150** into the surgical operation area. The extendable proximal part **112,** in retracted position, comprises a contracting and extensible micro-spring **113,** which enables the proximal part to extend and contract. The solution resistant adhesive patch **140** comprises a central tiltable collar **142** around the central opening of the adhesive patch.

Figure Fig. 3b illustrates an embodiment of the soft robotic imaging device **100,** wherein the contracting and extensible micro-spring **113** is in extended position. In the embodiment, the soft robotic imaging device 100 further comprises locking means **128** at the handle **160** side of the proximal part **112.**

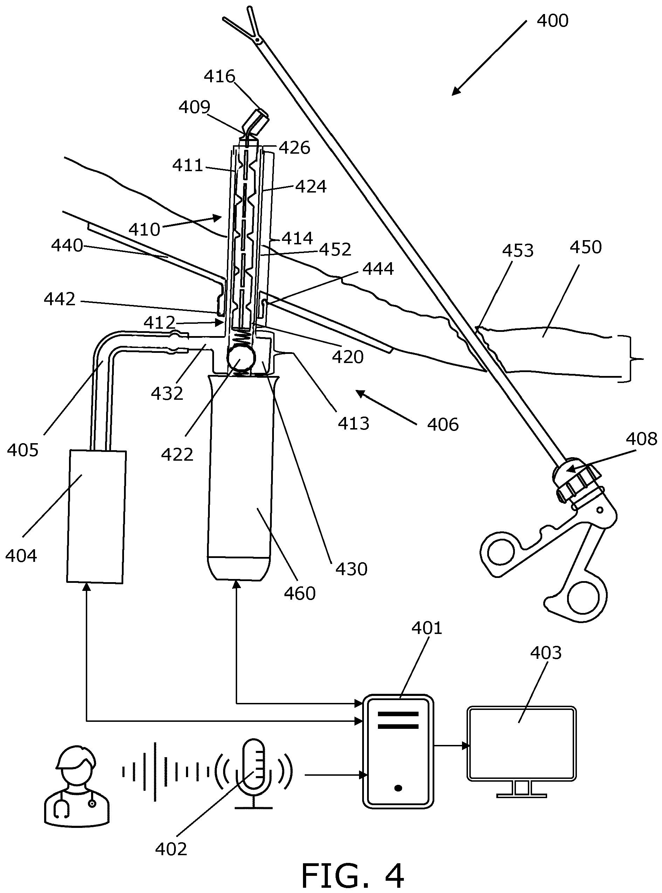

Referring to Fig. 4 there is shown a front view of an imaging system 400 for minimally invasive (laparoscopic) surgical procedure. The imaging system **400** comprises a soft robotic imaging device **406,** a computing device **401,** hands-free control system of the soft robotic imaging device **402,** an imagery display device **403** and a pump (i.e., a laparoscopic CO2 insufflator) **404** of a gas pressure control, which is connected to an inlet **432** of the trocar **420** by a hose **405.** The soft robotic imaging device **406** is inserted through a small incision **452** of the skin **450.** The soft robotic imaging device **406** for minimally invasive surgical procedure comprises a scope **410** arranged to be at least partially extendable and having a proximal part **412** and a soft robotic actuated flexible distal part **414** adapted to bend in multiple directions, a camera **416** with a light source positioned at an end of the flexible distal part of the scope, a laparoscopic trocar **420** comprising a gas channel **430** with an inlet **432** and is detachably connectable with the proximal part **412** of the scope **410,** a solution resistant adhesive patch **440** of the surgical access device to hold the trocar **420** on a skin **450** and a handle **460** attached to the end of the proximal part of the scope and arranged to accommodate the integrated electronic hardware.

The scope **410** comprises scope sleeve **411.** The proximal part **412** of the scope comprises a contracting and extensible micro-spring **413,** shown in contracted position. The soft robotic actuated flexible distal part **414** is in the form of cross-leaf structured flexible actuator with notches. The trocar **420** further comprises a trocar release button **422** and a trocar sleeve **424** surrounding a central sleeve of endoscope **411.** Between the sleeve **424** and sleeve of endoscope there is formed a gas channel **426.** The solution resistant adhesive patch **440** comprises a central tiltable collar **442** around the central opening of the adhesive patch, forming a tiltable part **444** of the patch between the collar and the patch.

The soft robotic actuated flexible distal part of the scope has two ends, at one end there is an imaging device (e.g., a camera) **416** attached, the second end is connected with the proximal part. The soft robotic actuated flexible distal part of the scope comprises multiple segments. Each segment in the cross-leaf structure is arranged to bend and rotate independently. As the extendable proximal part **412** is in retracted position, bending point **409** is formed to the segment which reaches out from the trocar sleeve. Flexibility of the scope enables it to bend around the bending point **409.** Through a second incision **453** of the skin **450** a surgical instrument **408,** e.g., a laparoscopic gasper is inserted through the skin into the surgical operation area, wherein a tip of the soft robotic actuated flexible distal part **414** with camera **416** is automatically bent towards the laparoscopic gasper to record the location and movements of the surgical instrument.

## Claims

1. A soft robotic imaging device for minimally invasive surgical procedure, the imaging device comprises
- a scope arranged to be at least partially extendable and having a proximal part and a soft robotic actuated flexible distal part adapted to bend in multiple directions;
- an imaging means positioned at an end of the flexible distal part of the scope;
- a surgical access device comprising a fluid channel and is detachably connectable with the proximal part of the scope;
- a fixation mechanism of the surgical access device; and
- an integrated electronic hardware.

2. The soft robotic imaging device according to claim 1, wherein the soft robotic actuated flexible distal part is constructed using one or more conductive polymers.

3. The soft robotic imaging device according to claim 2, wherein the one or more conductive polymers is selected from a group comprising polypyrrole (PPy), poly(3,4-ethylenedioxythiophene) polystyrene sulfonate (PEDOT:PSS), polyaniline, polythiophene or polyacetylene, poly(3-hexylthiophene) (P3HT).

4. The soft robotic imaging device according to any of the preceding claims, wherein the imaging means is configured to capture and transmit video imagery of surrounding tissues during the minimally invasive surgical procedure.

5. The soft robotic imaging device according to any of the preceding claims, wherein the soft robotic imaging device comprises a handle attached to the end of the proximal part of the scope arranged to accommodate the integrated electronic hardware.

6. The soft robotic imaging device according to any of the preceding claims, wherein the flexible distal part is cross-leaf structured with notches, chain-like structured, tentacle-structured, tube-structured, sheet-structured, amorphous or muscle-like.

7. The soft robotic imaging device according to any of the preceding claims, wherein the soft robotic actuated flexible distal part comprises biocompatible encapsulation.

8. The soft robotic imaging device according to any of the preceding claims, wherein the fixation mechanism is constructed of a biocompatible and antiallergic adhesive polymer.

9. The soft robotic imaging device according to any of the preceding claims, wherein the imaging means comprises one or more of a light source, an anti-fog coating, one or more additional sensors.

10. The soft robotic imaging device according to any of the preceding claims, wherein the electronic hardware comprises a microprocessor selected from a group comprising a vision processing unit (VPU), computer vision processor (CVP) chip, system-on-chip (SOC) or Complementary Metal-Oxide-Semiconductor (CMOS) chip.

11. An imaging system for minimally invasive surgical procedure, wherein the system comprises
- a soft robotic imaging device according to any of claims 1-10;
- a computing device comprising an automated control system of the soft robotic imaging device arranged to receive and process imagery from the soft robotic imaging device;
- a hands-free control system of the soft robotic imaging device;
- an imagery display device arranged to display the imagery received from the soft robotic imaging device;
- a first connectivity interface configured to establish communication between the soft robotic imaging device and the computing device; and
- a second connectivity interface configured to establish communication between the hands-free control system and the soft robotic imaging device.

12. The surgery system according to claim 11, wherein the automated control system is arranged, based on the processed imagery, to
- detect surgical instruments;
- detect and visualise surgical operation area;
- monitor condition of surrounding tissues and upon detection of tissue damages to generate an alert related to the detected tissue damages;
- track movements of surgical instruments during the minimally invasive surgical procedure;
- synchronize the movements with surgical instruments.

13. The surgery system according to claim 11 or 12, wherein the hands-free control system is arranged to trigger and to guide actions of the soft robotic imaging device.

14. A controlling method of a soft robotic imaging device and an imaging system for minimally invasive surgical procedure, wherein the method comprises steps of
- receiving, in a computing device, imagery from an imaging means positioned at an end of a flexible distal part of a scope at a site of the minimally invasive surgical procedure;
- collecting data by the computing device from the received imagery by detecting surgical instruments, detecting features of surroundings of the surgical instruments, detecting and visualising surgical operation area,
- monitoring condition of surrounding tissues and upon detection of tissue damages generating an alert related to the detected tissue damages, and tracking movements of surgical instruments during the MIS procedure;
- based on the collected data, synchronizing movements of the flexible distal part of the scope with surgical instruments.

15. The controlling method according to claim 14, wherein the synchronizing movements comprise applying voltage to the soft robotic actuated flexible distal part of the scope.
